# EUROPÄISCHE PATENTSCHRIFT

(11) **EP 2 237 782 B1**
(45) Veröffentlichungstag und Bekanntmachung des Hinweises auf die Patenterteilung: **11.04.2018**
(21) Anmeldenummer: 09706015.6
(22) Anmeldetag: 30.01.2009
(51) Int. Cl.: A61K 31/495, A61P 29/00

(54) **VERWENDUNG VON TETRAHYDROPYRIMIDINEN**
USE OF TETRAHYDROPYRIMIDINES
UTILISATION DE TÉTRAHYDROPYRIMIDINES

(30) Priorität: 30.01.2008 DE 102008006780
(43) Veröffentlichungstag der Anmeldung: 13.10.2010
(73) Patentinhaber: bitop AG, 58453 Witten (DE)
(72) Erfinder: TOLBA, Rene, 52074 Aachen (DE); VON ECHTEN-DECKERT, Gerhild, 53177 Bonn (DE); LENTZEN, Georg, 46483 Wesel (DE); BILSTEIN, Andreas, 50129 Bergheim (DE)
(74) Vertreter: Schneiders, Josef
(86) Internationale Anmeldenummer: PCT/EP2009/000641
(87) Internationale Veröffentlichungsnummer: WO 2009/095269

(56) Entgegenhaltungen:
- DE-A1- 10 006 578
- DE-A1-102004 016 129
- US-B1- 6 267 973
- PECH THOMAS ET AL: "A natural tetrahydropyrimidine, ectoine, ameliorates ischemia reperfusion injury after intestinal transplantation in rats.", PATHOBIOLOGY : JOURNAL OF IMMUNOPATHOLOGY, MOLECULAR AND CELLULAR BIOLOGY 2013, vol. 80, no. 2, 27 October 2013 (2013-10-27), pages 102-110, ISSN: 1423-0291

## Beschreibung

Die Erfindung betrifft Tetrahydropyrimidine und ihre Verwendung bei der Vorbeugung und Behandlung von postoperativem Entzündungsstress und - schmerz.

Aus extremophilen Organismen isolierte niedermolekulare organische chemische Verbindungen haben einen bemerkenswerten Einfluss auf biologische Systeme und Strukturen. Solche Verbindungen werden als Osmolyte oder kompatible Solute bezeichnet und haben inzwischen in zahlreiche kosmetische Präparate Eingang gefunden.

Zu den kompatiblen Soluten gehören neben Zuckern, Schwefelverbindungen, Polyolen und Aminosäuren insbesondere auch Tetrahydropyrimidinderivate, wie Ectoin und Hydroxyectoin. Sie werden von extremophilen Mikroorganismen unter Stressbedingungen synthetisiert und dienen der Stabilität der Zellstrukturen dieser Mikroorganismen unter extremen thermischen, chemischen und physikalischen Bedingungen. Ein Beispiel hierfür sind halophile Mikroorganismen, die sich dem wechselnden Salzgehalt in salzhaltiger Umgebung anpassen müssen und darin überleben müssen.

Von Ectoin und seinen Derivaten ist die stabilisierende Wirkung auf Protein- und Nukleinsäurestrukturen bekannt, die zur Stabilisierung von biologischem Material und Arzneimittelzubereitungen herangezogen werden kann.

Für Ectoin und seine Derivate wurden zwischenzeitlich eine Reihe von medizinischen Anwendungen beschrieben, so beispielsweise die Verwendung als Dermatologikum wie auch zur Effektivitätssteigerung Protein enthaltender Wirkstoffe. Nachgewiesen wurde ferner eine therapeutische Wirkung bei der Behandlung von Endothelfunktionsstörungen. Das US-Patent US 6,267,973 B1 beschreibt die Wirkung von Ectoin als Moisturizer, d. h. als Befeuchtungsmittel der Haut. In der DE 10 2004 016 129 A1 wird die Stabilisierung von Nachtkerzenöl mit Hilfe von Ectoin offenbart. Das Dokument DE 100 06 578 A1 befasst sich mit der Verwendung von kompatiblen Soluten wie Ectoin als Inhibitoren des enzymatischen Abbaus von makromolekularen Biopolymeren.

Angesichts der bekannten physiologischen Wirkungen von Ectoin ist die jetzt gefundene Wirksamkeit bei postoperativem Entzündungsstress und -schmerz, die häufig als Folge von operativen Eingriffen insbesondere im Bauchraum auftreten, absolut überraschend.

Entsprechend betrifft die Erfindung Tetrahydropyrimidine der Formel 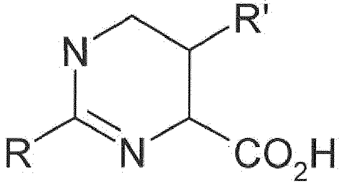 worin R ein Wasserstoffatom oder ein Kohlenwasserstoffrest mit bis zu acht Kohlenstoffatomen ist, R' Wasserstoff, OH oder OR" ist und R" R oder COR ist, wobei R die oben angegebene Bedeutung hat, zur Verwendung bei der Vorbeugung oder Behandlung von durch mechanische Belastungen bei operativen Eingriffen hervorgerufenem postoperativen Entzündungsstress und -schmerz, insbesondere im Magen-/Darmbereich, ausgenommen transplantationsbedingtem Stress und/oder Schmerz.

Operationen oder Eingriff im Sinne der Erfindung ist jeder Eingriff, der zu einer mechanischen Belastung des Körpers führt, beispielsweise auch bei Endoskopuntersuchungen oder auch Spiegelungen im Gelenkbereich oder im Bauchraum. Insbesondere sind aber Eingriffe operative Eingriffe, die größer sind als die nur punktförmige Öffnung/Durchtrennung der Haut.

Bei operativen Eingriffen kommt es häufig zu sogenanntem postoperativen Entzündungsstress und -schmerz, die dem Patienten erhebliche Probleme bereiten können. Dieser Entzündungsstress und -schmerz ist häufig genug gar nicht mit dem eigentlichen Eingriff verbunden, sondern beruht auf Entzündungserscheinungen, die eine Folge der mechanischen Belastung von bei dem Eingriff selbst nicht betroffenen Teilen sind, etwa bei Eingriffen im Bauchraum, insbesondere im Magen-/Darmbereich, aber auch bei Eingriffen bei Leber und Nieren sowie bei Endoskopuntersuchungen. Eine solche mechanische Belastung ergibt sich beispielsweise für den Bauchraum aus der Notwendigkeit, während des Eingriffs Darmschlingen zu verlagern, den Bauchraum zu erweitern oder, insbesondere bei Untersuchungen, den Darm selbst oder den Bauchraum mit Druck zu beaufschlagen. Die daraus resultierenden Entzündungserscheinungen und der damit verbundene Entzündungsstress und -schmerz dauern zuweilen auch noch lange nach dem Eingriff an. Dies gilt entsprechend für Eingriffe in anderen Körperregionen, etwa auch für Zahnextraktionen, Kieferoperationen oder Operationen im Zusammenhang mit Frakturen.

Ectoine im Sinne der Erfindung sind (4S)-1,4,5,6-Tetrahydropyrimidin-4-carbonsäuren und ihre Derivate. Die Tetrahydropyrimidincarbonsäuren können in der 2-Position eine Kohlenwasserstoffgruppe mit bis zu acht Kohlenstoffatomen aufweisen, etwa eine Alkylgruppe oder eine Methylgruppe. Das Tetrahydropyrimidin kann ferner in der 5-Position durch eine Hydroxygruppe substituiert sein, insbesondere durch eine (5S)-Hydroxygruppe. Die Hydroxygruppe kann pharmazeutisch verträglich verethert oder verestert sein.

Bevorzugte Ectoine sind Ectoin selbst, (4S)-2-Methyl-1,4,5,6-tetrahydropyrimidin-4-carbonsäure, und Hydroxyectoin (4S, 5S)-5-Hydroxy-2-methyl-1,4,5,6- tetrahydropyrimidin-4-carbonsäure.

Erfindungsgemäß können mehrere Ectoine zusammen eingesetzt werden. Als Derivate kommen solche in Frage, die im Wesentlichen die gleichen oder bessere Wirkungen als die entsprechende Basissubstanz aufweisen.

Die erfindungsgemäßen Tetrahydropyrimidine können selbstverständlich mit üblichen Adjuvantien und Hilfsstoffen kombiniert werden. Die zum Einsatz kommenden Mittel können für die orale oder parenterale Verabreichung vorgesehen sein, beispielsweise als Tablette, Kapsel oder als Lösung. Es kann in Dosen von 1 bis 250 mg pro kg Körpergewicht und Tag, vorzugsweise 5 bis 150 mg, und insbesondere 10 bis 100 mg, verabreicht werden, in einer oder mehreren täglichen Dosen.

Neben der oralen oder parenteralen Verabreichung kann auch eine topische Verabreichung vorgenommen werden, beispielsweise in Form einer Spülung des zu behandelnden Darmbereichs oder des Bauchraums mit einer pharmazeutisch verträglichen Lösung, etwa einer physiologischen Lösung, die das Tetrahydropyrimidin enthält. Aufgrund der außerordentlich guten Körperverträglichkeit der Tetrahydropyrimidine, die voll wasserlöslich sind, können solche Spüllösungen bis zu 25 % des Tetrahydropyrimidins enthalten. In der Regel wird der Gehalt allerdings bei 0,5 bis 5 % liegen.

Die erfindungsgemäßen Tetrahydropyrimidine eignen sich insbesondere auch zur Verabreichung und Verwendung zusammen mit der Polysol-Lösung der Firma Doorzand Medical Innovations, die zur Spülung und Konservierung auch von Transplantaten eingesetzt wird. Der Polysol-Lösung kann 0,01 bis 10 Gew.-% Tetrahydropyrimidin zugesetzt werden, insbesondere etwa 0,1 bis 1 %.

Die Tetrahydropyrimidine können auf breiter Basis eingesetzt werden, bei Eingriffen in praktisch allen Körperzonen und bei praktisch allen Operationsformen. Dies gilt bis hin zu Zahnextraktionen, Kieferoperationen und Implantationen, auch von Zähnen, künstlichen Gelenken, bis hin zu Augenoperationen. Erfindungsgemäß ausgenommen sind Transplantationen.

Zur näheren Untersuchung wurde in einem Tiermodell bei Ratten eine Darmentzündung durch Verabreichung von 10 mg/kg Trinitrobenzolsulfonsäure in 50 % Ethanol unter leichter Ethernarkose induziert. Es entwickelten sich Läsionen, die vier Tage später makroskopisch untersucht wurden. Ectoin wurde oral in verschiedenen Dosen (30 bis 300 mg/kg) eine Woche vor der Verabreichung von TNBS und vier Tage anschließend verabreicht. Ectoin war in der Lage, das Ausmaß der Läsionen zu vermindern, wobei mit einer Dosis von 100 mg/kg eine maximale Wirkung beobachtet werden konnte.

Biochemisch gesehen verhinderte Ectoin Veränderungen in der Myeloperoxidaseaktivität und verminderte den Glutathionspiegel im Dickdarm. Ectoin schützte ferner vor Veränderungen im Spiegel verschiedener Mediatoren, unter Einschluss von IKAM-1, DNFα, IL-1β, IL-10, LTB₄ und PGE₂ sowohl im Blut als auch im Dickdarmgewebe. Die Wirkung war vergleichbar mit derjenigen von Sulfasalazin mit 300 mg/kg, das als Referenzwirkstoff verwandt wurde. Die Schutzwirkung von Ectoin konnte anhand der histopathologischen Untersuchung des Dickdarms bestätigt werden.

### Beispiel (nicht Gegenstand der Erfindung)

Ratten wurden unter Standardbedingungen Dünndarmabschnitte entnommen und sofort mit kalter Kochsalzlösung gespült, anschließend mit Polysol-Lösung (Fa. Doorzand Medical Innovations, Amsterdam/NL). Die entnommenen Dünndarmabschnitte wurden ischemisch 18 Stunden in 50 ml Nährlösung bei 4°C aufbewahrt, wobei der Polysollösung in der Hälfte der Fälle Ectoin (5 mg/kg) zugesetzt wurde.

Nach der Lagerung bei 4°C wurden die Dünndarmabschnitte in vitro bei 37°C mit modifiziertem KHB-Medium reperfurdiert (5 % Dextran 78, 0,95 % KHB, 0,37 g/L CaCl₂, 2 g/L Glucose, 0,6 g/L Dexamethason, 70 mg/L Atropin und 0,21 % Natriumbicarbonat). Zur Sauerstoffversorgung wurde eine Mischung aus 95 % O₂ und 5 % CO₂ verwandt. Der Sauerstoff-Partialdruck wurde kontinuierlich über 500 mmHg gehalten.

Am Ende der Reperfusion wurden alle Gewebeproben bei -80°C in flüssigem Stickstoff eingefroren und auf ihren Zustand, die Laktatdehydrogenase (LDH)-Freisetzung und die Nitritfreisetzung als Maß für NO untersucht.

Für die LDH-Freisetzung als Maß für die Gewebequalität wurde für die in Polysol konservierten Proben Werte von 47,4 +/-12,21 U/I und 54,5 +/-8,57 U/I nach 15 und 30 min gemessen. Für mit Ectoin modifiziertes Polysol ergaben sich 8,8 +/- 3,42 U/I nach 15 min und 25,4 +/- 8,2 U/I nach 30 min. Die Verwendung von Ectoin reduziert somit das Ausmaß der Gewebeschädigung, was sich positiv auf den Heilungsprozess nach Operationen und Transplantationen auswirken sollte.

Die NOₓ-Bildung gilt gemeinhin als Indikator für (oxidativen) Stress, unter dem eine Gewebeprobe steht.

In der mit Polysol behandelten Kontrollgruppe ergab sich eine NOₓ-Bildung von 0,78 +/- 0,063 mmol/L, während in den mit Ectoin dotierten Kontrollgruppen der gemessene Wert 0,325 +/-1,05 mmol/L betrug. Die Halbierung an NOx- steht für eine Halbierung der NO-Bildung und damit für eine erhebliche Verminderung des oxidativen Stresses.

Die mikroskopischen Untersuchungen zeigen an den mit Ectoin behandelten Gewebeproben eine deutliche Verbesserung des Zustandes der Mikrovilli gegenüber den nur mit Polysol behandelten Proben.

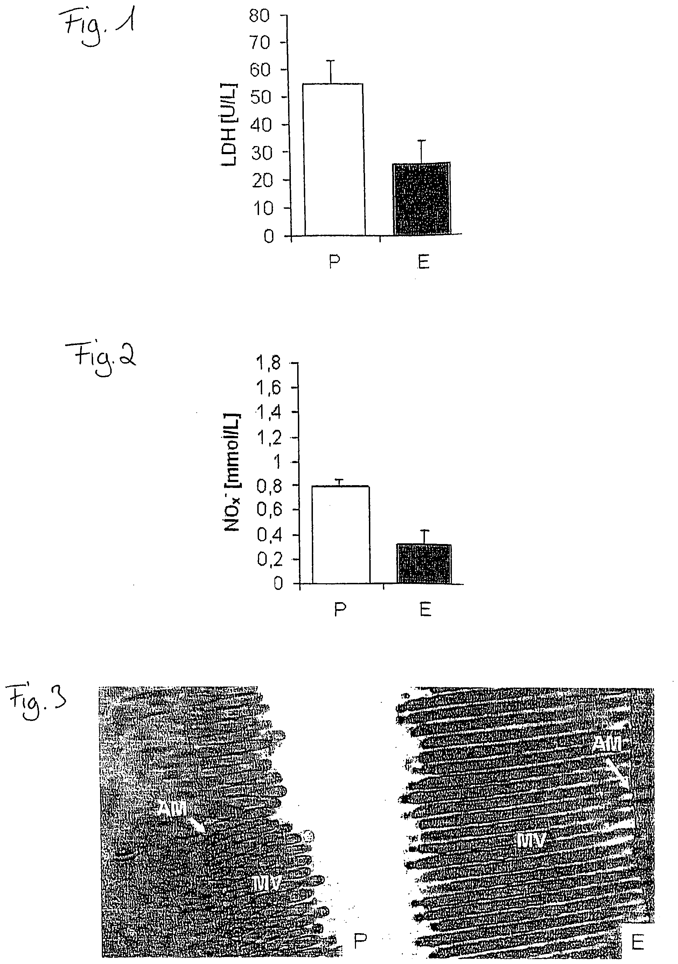

Fig. 1 zeigt die Werte für die Laktatdehydrogenase (LDH)-Freisetzung für mit Polysol allein (P) und unter Zusatz von Ectoin (E) in U/L.
Fig. 2 zeigt die NOₓ⁻Freisetzung in mmol/L für mit Polysol (P) und mit Ectoin (E) behandelte Proben.
Fig. 3 zeigt elektronenmikroskopische Aufnahmen von Gewebeproben nach 30 minütiger Reperfusion mit Sauerstoff in Polysol (P) und mit Ectoin (E). AM zeigt die applikale Zellmembran, MV die Mikrovilli, die in mit Ectoin behandelten Proben in deutlich besserem Zustand sind.

## Patentansprüche

1. Tetrahydropyrimidine der Formel 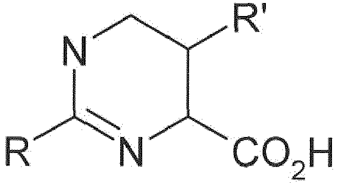 worin R ein Wasserstoffatom oder ein Kohlenwasserstoffrest mit bis zu acht Kohlenstoffatomen ist, R' Wasserstoff, OH oder OR" ist und R" R oder COR ist, wobei R die oben angegebene Bedeutung hat,
zur Verwendung in einem Verfahren zur Vorbeugung oder Behandlung von durch mechanische Belastungen bei operativen Eingriffen hervorgerufenem postoperativen Entzündungsstress und/oder -schmerz, ausgenommen transplantationsbedingtem Stress und/oder Schmerz.

2. Tetrahydropyrimidin zur Verwendung nach Anspruch 1 zur Behandlung von Entzündungsstress und/oder -schmerz, der durch Eingriffe im Bauchraum, an der Leber oder an den Nieren hervorgerufen wird.

3. Tetrahydropyrimidin zur Verwendung nach Anspruch 2 zur Behandlung entzündungsbedingter Schmerzen im Magen-/Darmbereich.

4. Tetrahydropyrimidin zur Verwendung nach Anspruch 1 zur Behandlung von entzündungsbedingtem Stress und/oder Schmerz nach Endoskopanwendung, Spiegelungen im Gelenkbereich oder laparoskopischen Eingriffen.

5. Tetrahydropyrimidin zur Verwendung nach Anspruch 1 zur Behandlung von entzündungsbedingtem Stress und/oder Schmerz aufgrund von Zahnextraktionen, Kieferoperationen, Operationen im Zusammenhang mit Frakturen, Implantationen oder Augenoperationen.

6. Tetrahydropyrimidin zur Verwendung nach einem der Ansprüche 1 bis 5, **dadurch gekennzeichnet, dass** R Methyl ist.

7. Tetrahydropyrimidin zur Verwendung nach einem der Ansprüche 1 bis 6, **dadurch gekennzeichnet, dass** R' Hydroxy ist.

8. Tetrahydropyrimidin zur Verwendung nach einem der Ansprüche 1 bis 7, **dadurch gekennzeichnet, dass** das Tetrahydropyrimidin Ectoin oder Hydroxyectoin ist.

9. Mittel enthaltend ein Tetrahydropyrimidin zur Verwendung nach einem der Ansprüche 1 bis 8 in einer Menge von 10 bis 10.000, vorzugsweise 20 bis 1.000 und besonders bevorzugt 50 bis 500 mg.

10. Lösung zur topischen Anwendung, enthaltend ein Tetrahydropyrimidin zur Verwendung nach einem der Ansprüche 1 bis 9 in einer physiologisch verträglichen Form in einer Menge von 0,1 bis 25 Gew.-%, vorzugsweise von 0,5 bis 5 Gew.-%.

## Claims

1. Tetrahydropyrimidines of the formula 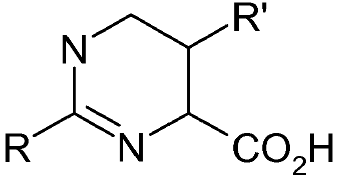 where R is a hydrogen atom or a hydrocarbon moiety comprising up to eight carbon atoms, R' is hydrogen, OH or OR" and R" is R or COR, with R having the above described meaning,
for use in a method for prevention or treatment of postoperative inflammatory stress and/or pain caused by mechanical impacts in surgical interventions, with the exception of transplantation-related stress and/or pain.

2. Tetrahydropyrimidine for use according to claim 1 for the treatment of inflammation-related stress and/or pain caused by interventions in the abdomen, the liver, or the kidneys.

3. Tetrahydropyrimidine for use according to claim 2 for the treatment of inflammation-related pain in the gastrointestinal region.

4. Tetrahydropyrimidine for use according to claim 1 for the treatment of inflammation-related stress and/or pain after endoscopic interventions, joint endoscopy, or laparoscopic interventions.

5. Tetrahydropyrimidine for use according to claim 1 for the treatment of inflammation-related stress and/or pain caused by dental extractions, jaw surgery, surgeries in connection with fractures, implantations, or eye surgeries.

6. Tetrahydropyrimidine for use according to any of claims 1 to 5, **characterized in that** R is methyl.

7. Tetrahydropyrimidine for use according to any of claims 1 to 6, **characterized in that** R' is hydroxy.

8. Tetrahydropyrimidine for use according to any of claims 1 to 7, **characterized in that** the tetrahydropyrimidine is ectoine or hydroxyectoine.

9. Agent comprising a tetrahydropyrimidine for use according to any of claims 1 to 8 in an amount ranging between 10 and 10,000, preferably 20 and 1,000 and especially preferred between 50 and 500 mg.

10. Solution for topical application, comprising a tetrahydropyrimidine for use according to any of claims 1 to 9 in a physiologically compatible form in an amount ranging between 0.1 and 25 % w/w, preferably between 0.5 and 5 % w/w.

## Revendications

1. Tétrahydropyrimidines de formule 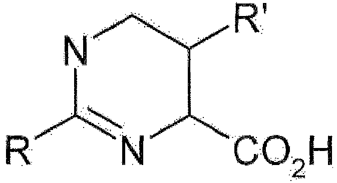 dans laquelle R représente un atome d'hydrogène ou un radical hydrocarboné comprenant jusqu'à huit atomes de carbone, R' représente hydrogène, OH ou OR" et R" représente R ou COR, R présentant la signification susmentionnée,
pour une utilisation dans un procédé pour la prévention ou le traitement du stress et/ou de la douleur inflammatoire(s) postopératoire(s), provoqué(e)(s) par des sollicitations mécaniques lors d'interventions opératoires, à l'exception du stress et/ou de la douleur provoqué(e)(s) par une transplantation.

2. Tétrahydropyrimidine pour une utilisation selon la revendication 1 pour le traitement du stress et/ou de la douleur inflammatoire (s) qui est/sont provoqué(e)(s) par des interventions dans l'abdomen, sur le foie ou sur les reins.

3. Tétrahydropyrimidine pour une utilisation selon la revendication 2 pour le traitement de douleurs inflammatoires dans la région de l'estomac/des intestins.

4. Tétrahydropyrimidine pour une utilisation selon la revendication 1 pour le traitement du stress et/ou de la douleur provoqué(e)(s) par une inflammation après un usage endoscopique, des scopies dans une zone articulaire ou des interventions par laparoscopie.

5. Tétrahydropyrimidine pour une utilisation selon la revendication 1 pour le traitement du stress et/ou de la douleur provoqué(e)(s) par une inflammation en raison d'extractions dentaires, d'opérations sur les mâchoires, d'opérations associées à des fractures, des implantations ou des opérations oculaires.

6. Tétrahydropyrimidine pour une utilisation selon l'une quelconque des revendications 1 à 5, **caractérisée en ce que** R représente méthyle.

7. Tétrahydropyrimidine pour une utilisation selon l'une quelconque des revendications 1 à 6, **caractérisée en ce que** R' représente hydroxy.

8. Tétrahydropyrimidine pour une utilisation selon l'une quelconque des revendications 1 à 7, **caractérisée en ce que** la tétrahydropyrimidine est l'ectoïne ou l'hydroxyectoïne.

9. Agent contenant une tétrahydropyrimidine pour une utilisation selon l'une quelconque des revendications 1 à 8 en une quantité de 10 à 10.000, de préférence de 20 à 1000 et de manière particulièrement préférée de 50 à 500 mg.

10. Solution pour une application topique, contenant une tétrahydropyrimidine pour une utilisation selon l'une quelconque des revendications 1 à 9 sous une forme physiologiquement acceptable en une quantité de 0,1 à 25% en poids, de préférence de 0,5 à 5% en poids.
